# EUROPÄISCHE PATENTSCHRIFT

(11) **EP 1 325 103 B1**
(45) Veröffentlichungstag und Bekanntmachung des Hinweises auf die Patenterteilung: **01.03.2006**
(21) Anmeldenummer: 01965164.5
(22) Anmeldetag: 02.08.2001
(51) Int. Cl.: C11D 11/00, C11D 17/00, C11D 3/20, A61L 2/18, C11D 3/00

(54) **HAFTENDES SANITÄRREINIGUNGS- UND BEDUFTUNGSMITTEL**
ADHESIVE SANITARY CLEANING AND DEODORISING PRODUCT
AGENT SANITAIRE ET DESODORISANT ADHESIF

(30) Priorität: 29.09.2000 DE 10048887
(43) Veröffentlichungstag der Anmeldung: 09.07.2003
(73) Patentinhaber: Buck-Chemie GmbH, 71083 Herrenberg (DE)
(72) Erfinder: DETTINGER, Johannes, 71260 Horb a. N. (DE); JAESCHKE, Edgar, 70794 Filderstadt (DE); SEIDEL, Detlef, 71131 Jettingen (DE)
(74) Vertreter: Mammel, Ulrike
(86) Internationale Anmeldenummer: PCT/EP2001/008972
(87) Internationale Veröffentlichungsnummer: WO 2002/026925

(56) Entgegenhaltungen:
- EP-A- 1 029 911
- WO-A-01/19944
- WO-A-99/66017
- DE-A- 19 715 872
- DE-A- 19 918 185
- US-A- 5 460 742
- US-A- 5 877 135
- US-A- 5 990 066

## Beschreibung

Die Erfindung betrifft ein haftendes Sanitärmittel zum Reinigen und/oder Desinfizieren und/oder zur Duftstoffabgabe für Sanitärgegenstände wie Toilettenspülbekken.

Diese Sanitärmittel sind viskose, im allgemeinen pastöse Gele, die aus einem entsprechenden Behältnis direkt auf der Oberfläche des Sanitärgegenstandes aufgebracht werden und dort haften und erst nach einer größeren Anzahl von Spülvorgängen abspülbar sind.

Durch die direkte Haftung des Mittels auf der Oberfläche des Sanitärgegenstandes ist es nicht erforderlich, zusätzlich Behältnisse wie die sogenannten "WC-Körbchen" vorzusehen, deren Benutzung vom Verbraucher insbesondere beim Ersetzen des Sanitärmittels und bei der Reinigung der Toilette als unhygienisch empfunden wird.

Solche haftenden Sanitärmittel sind aus der WO 99/66017 bekannt. Sie umfassen neben Tensiden, Wasser und Duftstoffen auch Haftvermittler und sind viskose, feste oder pastöse Mittel. Auch ist aus der WO99/66017 bekannt, daß rigide Gele zusätzlich Glykole und Glykolderivate umfassen können.

Die bekannten haftenden Sanitärmittel lassen sich auf einfache und hygienische Art und Weise mit einer geeigneten Vorrichtung applizieren, haften an der Oberfläche des Sanitärgegenstands, behalten ihre Form bei und werden selbst unter Einwirkung von Wasser nicht als Ganzes herabgespült, sondern erst nach einer Vielzahl von Spülungen vollständig aufgelöst.

Werden die bekannten haftenden Sanitärmittel allerdings auf Sanitärgegenständen mit geringer Spülfrequenz eingesetzt, so wurde festgestellt, daß die Oberfläche des Sanftärmittels grieselig, uneben und zerfurcht wird und sich durch Austrocknung weiß verfärbt. Dies ist ästhetisch nicht ansprechend und signalisiert dem Verbraucher ein funktionsuntüchtiges Mittel. Im Extremfall bildet sich darüber hinaus ein schwer löslicher Rückstand auf dem Sanitärgegenstand.

Der vorliegenden Erfindung liegt die Aufgabe zugrunde, ein haftendes Sanitärmittel bereitzustellen, das unabhängig von der Spülfrequenz der Toilette eine ansehnliche und glatte Oberfläche beibehält und bei dem die Bildung schwer löslicher Rückstände in Folge von Austrocknung vermieden wird.

Diese Aufgabe wird durch die Merkmale des Anspruchs 1 gelöst.

Es wurde festgestellt, daß bei einem aus Tensiden, einem Haftvermittler und Wasser bestehenden haftenden Sanitärmittel eine von der Spülfrequenz unabhängige glatte Oberfläche des aufgebrachten Mittels erreicht werden und die Bildung einer weißen verkrusteten Oberfläche von schwer löslichen Resten vermieden werden kann, wenn das Mittel zusätzlich entweder wenigstens eine aus der Gruppe Glycerin, 1,3-Dihydroxypropan, 1,3- oder 1,4- Dihydroxybutan, 1,3-Dihydroxyisobutan, Pentaerythrit ausgewählte Verbindung oder zwischen 1 und 20 Gew.%, vorzugsweise zwischen 5 und 15 Gew.% und besonders bevorzugt zwischen 7 und 13 Gew.% einer aliphatischen Di-, Oligo- oder Polyhydroxyverbindung oder deren Ether umfaßt.

Hierdurch wird die Feuchtigkeit der Oberfläche des haftenden Sanitärmittels erhalten, so daß das aufgebrachte Mittel auch bei einer geringen Spülfrequenz eine glatte, im wesentlichen transparente Oberfläche beibehält. Durch die Zugabe des erfindungsgemäßen Feuchthaltemittels wird zudem die bei den bekannten Mitteln beobachtete Vertrocknung der Oberfläche mit der Bildung schwer löslicher Reste vermieden.

Der Einsatz der erfindungsgemäßen Verbindungen zu den haftenden Sanitärmitteln bewirkt gleichzeitig eine positive Beeinflussung der Lebensdauer des Mittels, denn diese Verbindungen verbessern das Auflöseverhalten des Sanitärmittels und mindern somit die erforderliche Menge an einzusetzendem Parfüm. Bei 5 ml aufgetragener Masse in einem Strang von 1,3 cm Breite werden mit dem erfindungsgemäßen Mittel Spülzahlen von 100 bis 150 Spülungen gegenüber Spülzahlen von bis zu 300 Spülungen bei den bekannten haftenden Sanitärmitteln erreicht. Spülzahlen von 100 bis 150 sind wünschenswert, da das Mittel dann in der Regel bei einem 3 bis 4 Personenhaushalt einmal pro Woche zu erneuern ist und zudem bei einer Auflösung von 1/100 bis 1/150 des Mittels pro Spülvorgang die einzelnen Wirkstoffe des Mittels (einschließlich Parfüm) in der gewünschten Konzentration abgegeben werden. Werden - wie bei den bekannten Mitteln - Spülzeiten von bis zu 300 Spülungen erhalten, muß die Konzentration des Parfüms erhöht werden, um pro Spülung denselben Dufteindruck zu erhalten.

Die erfindungsgemäßen Feuchthaltemittel dienen gleichzeitig als Lösungsvermittler und bewirken eine Beschleunigung des Auflösevorganges des Mittels und damit eine konstante Freisetzung der einzelnen Bestandteile des Sanitärmittels in konzentrierter Form bei jedem Spülvorgang. Das Abspülverhalten des Mittels wird bei Verwendung der erfindungsgemäßen Feuchthaltemittel gegenüber der Verwendung von Natriumsulfat, das beispielsweise in der WO 99/66017 eingesetzt wird, deutlich verbessert. Auch auf die Konsistenz des Mittels haben die erfindungsgemäßen Feuchthaltemittel einen positiven Einfluß.

Als aliphatische Di-, Oligo- oder Polyhydroxyverbindung oder deren Ether kann jede aliphatische Verbindung eingesetzt werden, die wenigstens zwei Hydroxygruppen aufweist. Beispiele für aliphatische Dihydroxyverbindungen sind unter anderem Glykol, 1,2- oder 1,3 Dihydroxypropan, 1,2-, 1,3-, 2,3- oder 1,4-Dihydroxybutan, die Isomeren des Dihydroxyisobutans, die Dihydroxypentane etc.

Auch Trihydroxyverbindungen, wie beispielsweise Glycerin oder auch längerkettige Trihydroxyverbindungen, wie beispielsweise Trihydroxyhexan, Trihydroxyhepten etc., können als Feuchthaltemittel eingesetzt werden.

Als Tetrahydoxyverbindung kann zum Beispiel Pentaerythrit eingesetzt werden.

Als weitere Oligohydroxyverbindungen kommen Pentahydroxy-, Hexahydroxy-Verbindungen etc. in Betracht. Auch Zucker, Polysaccharide, wie Stärke, Cellulose oder Polymere sind als Oligo- oder Polyhydroxyverbindungen einsetzbar.

Es ist ebenfalls möglich, als Feuchthaltemittel Ether der Di-, Oligo- oder Polyhydroxyverbindungen einzusetzen, wie beispielsweise Diethylenglykol, Oligoethylenglykole wie das Triethylenglykol, aber auch Polyhydroxyether wie beispielsweise Polymethylen- oder Polyethylenglykol.

Das erfindungsgemäße Feuchthaltemittel kann eine gerade, wie beispielsweise im Glykol, aber auch eine ungeradzahlige Anzahl von Kohlenstoffatomen, wie beispielsweise im Glycerin, aufweisen. Ebenfalls können unverzweigte Moleküle, wie beispielsweise Glykol, aber auch verzweigte Moleküle wie Pentaerythrit, als Feuchthaltemittel verwendet werden.

Um die gewünschte Feuchtigkeitsregulierung zu erreichen, weist das erfindungsgemäße Mittel zwischen 1 und 20 Gew.%, vorzugsweise zwischen 5 und 15 Gew.% und besonders bevorzugt zwischen 7 und 13 Gew.%, der aliphatischen Di-, Oligo- oder Polyhydroxyverbindungen oder deren Ether auf. Besonders bevorzugt ist, als Feuchthaltemittel Glycerin einzusetzen.

Der Haftvermittler bewirkt in Anwesenheit von Wasser, daß das Mittel an der Oberfläche haftet. Im allgemeinen bildet er netzwerkartige Strukturen aus, die dem Mittel selbst unter der starken Krafteinwirkung durch Spülwasser die erforderliche Formbeständigkeit verleihen.

Die Moleküle des Haftvermittlers sind länger- oder langkettige, im wesentlichen gestreckte Moleküle, die wenigstens teilweise hydrophil sind und somit wenigstens einen hydrophilen Rest oder eine hydrophile Gruppe umfassen, die mit Wasser wechselwirkt.

Vorzugsweise sollte es sich bei den Haftvermittlern um unverzweigte Moleküle handeln, um die gewünschte Netzwerkbildung zu ermöglichen.

Der Haftvermittler kann entweder insgesamt hydrophil sein oder auch teilweise hydrophil, teilweise hydrophob.

Als Haftvermittler können organische Moleküle mit einem hydrophilen und einem hydrophoben Ende eingesetzt werden. Als hydrophile Reste können beispielsweise Polyalkoxygruppen, vorzugsweise Polyethoxy-, Polypropoxy- oder Polybutoxy- oder auch gemischte Polyalkoxygruppen wie beispielsweise Poly(ethoxypropoxy)gruppen eingesetzt werden. Besonders bevorzugt ist, als hydrophiles Ende einen Polyethoxyrest, umfassend zwischen 15 und 55 Ethoxygruppen, vorzugsweise zwischen 25 und 45 und besonders bevorzugt 35 +/- 5 Ethoxygruppen, zu verwenden.

Als hydrophiles Ende können auch anionische Gruppen, beispielsweise Sulfonate, Carbonate oder Sulfate eingesetzt werden.

Auch Stearate, insbesondere Natrium oder Kaliumstearat, sind als Haftvermittler geeignet.

Sofern die Haftvermittlermoleküle auch ein hydrophobes Ende aufweisen, sind für den hydrophoben Rest insbesondere geradkettige Alkylreste geeignet, wobei insbesondere geradzahlige Alkylreste wegen der besseren biologischen Abbaubarkeit besonders bevorzugt sind. Um die gewünschte Netzwerkbildung der Haftvermittlermoleküle zu erreichen, sollten die Moleküle unverzweigt sein.

Werden als hydrophobe Reste Alkylgruppen ausgewählt, so sind Alkylreste mit wenigstens zwölf Kohlenstoffatomen bevorzugt. Besonders gute Ergebnisse wurden mit einer Alkylkettenlänge zwischen 16 und 30 Kohlenstoffatomen, insbesondere 16, 18, 20 und 22 Kohlenstoffatomen, erzielt.

Als hydrophobe Reste können die Haftvermittler auch Alkylbenzolreste umfassen, beispielsweise Dodecylbenzol oder ähnliche Reste, wie es bei dem Haftvermittler Natriumdodecylbenzolsulfonat der Fall ist.

Bevorzugte Haftvermittler sind Polyalkoxyalkane, vorzugsweise ein Gemisch aus Alkyl(C20-C20)-ethoxylat mit 35 EO beziehungsweise Alkyl(C22)-ethoxylat mit 35 EO und Alkyl(C16-C18)-ethoxylat mit 30 EO, aber auch Alkylsulfonate wie Natriumdodecylbenzolsulfonat oder Alkylcarbonate oder Alkylsulfate.

Die Verwendung eines Gemischs der Haftvermittler Alkyl(C22)-ethoxylat (35EO) und Alkyl(C16-C18)-ethoxylat (30EO) wirkt sich auf die gewünschte Spülzahl im Sinne einer Reduktion und die Standfestigkeit des Mittels positiv aus. Darüber hinaus wird durch die gemeinsame Verwendung dieser Haftvermittler eine einfache Verarbeitbarkeit des Mittels erreicht.

Mit Verringerung der Anzahl der Alkoxygruppen wird der Haftvermittler lipophiler, wodurch beispielsweise die Löslichkeit von Parfum und damit die Intensität der Beduftung erhöht werden kann.

Es können auch Moleküle als Haftvermittler eingesetzt werden, die in wäßrigen Systemen allgemein als Verdicker wirken, zum Beispiel hydrophile Substanzen.

Die einzusetzende Konzentration des Haftvermittlers ist von dessen Hydrophilie und dessen Netzwerkbildungsvermögen abhängig. Sie können beispielsweise 10 und 40 Gew.%, vorzugsweise zwischen 15 und 35 Gew.% und besonders bevorzugt zwischen 20 und 30 Gew.%, betragen, was insbesondere bei der Verwendung Polyalkoxyalkanen bevorzugt ist.

Um mit den Haftvermittlermolekülen durch Anlagerung von Wasser die gewünschte Anzahl von Klebestellen bereitzustellen, sollte Wasser wenigstens zu 20 Gew.%, vorzugsweise zwischen 35 und 65 Gew.%, insbesondere zwischen 40 und 60 Gew.%, in der Formulierung enthalten sein. Der Wasseranteil ist unter anderem von der Hydrophilie des eingesetzten Haftvermittlers abhängig.

Als Tenside können prinzipiell alle bekannten anionischen und/oder nichtionischen amphoteren Tenside eingesetzt werden.

Neben den erfindungsgemäßen Bestandteilen kann das Sanitärmittel weitere übliche Bestandteile umfassen, beispielsweise Parfumöl, Desinfektionsmittel, Konservierungsstoffe, wie zum Beispiels Isothiazolon-Derivate oder Schaumstabilisatoren, wie Kokosfettsäureamidopropylbetain oder Kokosfettsäureamidopropyldimethylaminoxid, Kokosfettsäuremono/diethanolamid oder Alkylpolyetherglycerolethersulfate, aber auch Farbstoffe und/oder Kalk- oder Urinstein lösende Substanzen, insbesondere Säuren.

Auch ist es möglich, dem erfindungsgemäßen Mittel Olefinsulfonate, Ethersulfate und/oder Tenside, insbesondere Säuremethyltauride als Schäumer mit Selbstreinigungswirkung zuzusetzen.

Der Formulierung können - falls gewünscht - auch Salze, wie beispielsweise Natriumsulfat, hinzugegeben werden, um die Auflösegeschwindigkeit zu erhöhen. Der Salzanteil kann bis zu 10 Gew.%, vorzugsweise bis zu 5 Gew.%, betragen.

Das erfindungsgemäße Mittel kann in hygienischer Weise ohne Berührung von mit dem WC-Becken verbundenen, möglicherweise verunreinigten Vorrichtungen aufgebracht und erneuert werden.

Ein wesentlicher Vorteil des erfindungsgemäßen Mittels besteht darin, daß es nach den Wünschen des Verbrauchers portionierbar ist. Wünscht der Verbraucher eine intensivere Beduftung oder wird die Toilette häufig benutzt, so kann entsprechend stärker dosiert werden.

Das erfindungsgemäße Mittel kann auch auf einfache Weise gleichzeitig an verschiedenen Stellen des Sanitärgegenstands appliziert werden, beispielsweise, um sowohl auf der rechten, als auch auf der linken Seite einer Toilettenschüssel eine gleichmäßige Reinigungswirkung zu erzielen.

Auch kann an einem Sanitärgegenstand an verschiedenen Orten das erfindungsgemäße Mittel in unterschiedlichen Zusammensetzungen appliziert werden. Dies ermöglicht beispielsweise, daß zwei untereinander unverträgliche Inhaltsstoffe, beispielsweise halogenfreisetzende Mittel und oxidationsempfindliche Parfümierungsstoffe, durch örtliche Trennung dennoch zu einer gemeinsamen Reinigung und/oder Beduftung der Toilette eingesetzt werden können.

Die erreichte Haftung ist an dem Sanitärgegenstand selbst bei einer Anbringung an einer senkrechten Fläche so gut, daß sich das Mittel sogar unter der zusätzlichen Krafteinwirkung von Spülwasserströmen nicht ablöst.

Die erfindungsgemäßen Sanitärmittel sind erst nach einer größeren Anzahl von Spülvorgängen abspülbar. Die Anzahl der Spülvorgänge richtet sich natürlich nach der Zusammensetzung des jeweiligen Sanitärmittels, der aufgebrachten Menge sowie der Geometrie des aufgebrachten Sanitärmittels.

Dem Mittel können auch Duft- und/oder Riechstoffe zugegeben werden, um die Raumluft zu verbessern, wobei auch daran gedacht ist, ausschließlich Duft- und Riechstoffmedien derart in eine Toilettenschüssel einzubringen. Das Sanitärmittel läßt sich auch für andere Sanitärgegenstände einsetzen wie Urinale, Pissoirs, aber auch für Handspülbecken oder dergleichen.

Vorzugsweise ist das erfindungsgemäße Mittel ein salbenartiges pastöses und/oder cremeartiges Gel. Die Gele sind im wesentlichen formstabil, so daß sie nicht "herunterlaufen" oder "tropfen".

Die Haftung und auch die Form der Gele bleibt trotz der durch die Wasserspülung einwirkenden erheblichen Kräfte (Reibung, Deformation ...) erhalten. Vorzugsweise umfassen diese pastösen Gele etwa 1 Gew.% bis 25 Gew.%, insbesondere 5 bis 15 Gew.%, Duftstoffe, 1 Gew.% bis 20 Gew.%, insbesondere 5 bis 10 Gew.%., anionische Tenside, 20 Gew.% bis 40 Gew.% Haftvermittler, zwischen 1 und 20 Gew.%, vorzugsweise zwischen 5 und 15 Gew.%, aliphatische Di-, Oligo- oder Polyhydroxyverbindungen oder deren Ether oder der Verbindung(en) aus der Gruppe Glycerin, 1,3-Dihydroxypropan, 1,3- oder 1,4- Dihydroxybutan; 1,3-Dihydroxyisobutan, Pentaerythrit, 0 Gew.% bis 1 Gew.% Verdicker und 0 Gew.% bis 5 Gew.%, vorzugsweise zwischen 0 und 1 Gew.%, Konservierungsmittel sowie etwa 35 bis 65 Gew.% Wasser.

In dieser bevorzugten Ausführungsform werden insbesondere α-Olefinsulfonate, Methyltauride und Ethersulfate als anionische Tenside verwendet.

Als anionisches Tensid, das gleichzeitig als Schäumer fungiert, hat sich insbesondere Alkyl(C12-C14)-polyethylenglykol-(2EO)-ethersulfat-triisopropanol-ammoniumsalz bewährt. Dieses anionische Tensid ist bevorzugt, da es im Gegensatz zu Olefinsulfonat oder Taurid auch in höheren Konzentrationen nicht ausläuft.

Alkyl(C16-C18)-ethoxylat (30 EO) und Alkyl(C22)-ethoxylat (35 EO) werden bevorzugt als Haftvermittler und nichtionische Tenside eingesetzt. Das Mittel umfaßt weiterhin vorzugsweise 0 bis 3 Gew.% Aminoxide, wie zum Beispiel Kokosfettsäureamidopropyldimethylaminoxid (35 %ig), das von der Firma Rewo/Goldschmidt als Schaumstabilisator unter der Bezeichnung Rewominox B204 erhältlich ist und 0 bis 2 Gew.% Verdicker, wie beispielsweise einem Polysaccharid, insbesondere einem Xanthan-Gum, das zum Beispiel von Rhodia unter der Bezeichnung Rhodopol T erhältlich ist. Als Konservierungsmittel können beispielsweise Isothiazolon-Derivate verwendet werden.

Die Gele lassen sich vorzugsweise über Tuben, vergleichbar den Zahnpastatuben, Spritzen oder Kartuschen in das Toilettenbecken einbringen und bleiben dort für eine Vielzahl von aufeinanderfolgenden Spülvorgängen haften.

Die an einem Haake-Viskosimeter, System Platte-Kegel, Sensor PK 5 1 °, Schergefälle von 25 s⁻¹ und 20°C zu bestimmenden Viskositäten dieser pastösen Gele sollten wenigstens 15.000 mPas, üblicherweise wenigstens 50.000 mPas, vorzugsweise wenigstens 90.000 mPas und besonders bevorzugt wenigstens 110.000 mPas, betragen.

Die Erfindung wird nachfolgend anhand eines Ausführungsbeispiels und eines Vergleichsversuchs beschrieben.

### 1. Rezeptur des erfindungsgemäßen haftenden Sanitärmittels mit Glycerin

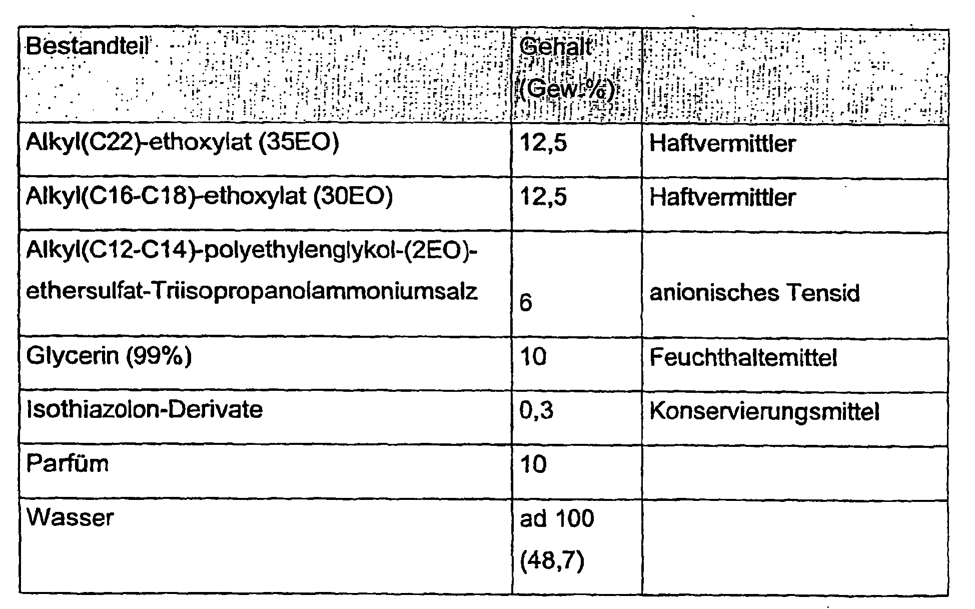

Die Viskosität beträgt bei 20° C circa 120.000 mPas (Haake-Viskosimeter, System Platte-Kegel, Sensor PK 51°, Schergefälle 25 s⁻¹).

Als Alkyl(C22)-ethoxylat (35EO) wurde das Imbentin V100 der Firma Dr. W. Kolb AG eingesetzt, als Alkyl(C16-C18)-ethoxylat (30EO) das Imbentin 168S/300 der Firma Dr. W. Kolb GmbH AG, als Alkyl(C12-C14)-polyethylenglykol-(2EO)-ethersulfat-Triisopropanolammoniumsalz das Marlinat 242/90 T der Firma CON-DEA GmbH, als Isothiazolon-Derivate das Parmetol K 40 der Firma Schülke & Mayr und als Parfüm das Citrix Extra F548373 der Firma Quest International.

Das erfindungsgemäße Mittel wies bis zur vollständigen Auflösung eine glatte und transparente Oberfläche des Gels auf, auch bei einer niedrigen Spülfrequenz von vier Spülungen pro Tag. Der Auflösevorgang war weitgehend unabhängig von der Spülfrequenz.

Beim Auftrag von 5 ml Masse mit einer Strangbreite von circa 1,3 cm wurden bei dem erfindungsgemäßen Mittel Spülzahlen zwischen 100 und 150 Spülungen ermittelt. Das Schaumvermögen von einem Gramm Mittel in einem Liter Wasser betrug 75/70/50 ml.

### Vergleichsversuche

Basierend auf der aus der WO 99/66017 bekannten Rahmenrezeptur wurden ein herkömmliches haftendes Sanitärmittel als Vergleich hergestellt und die Veränderung der Oberfläche des aufgetragenen Sanitärmittels bei geringen Spülfrequenzen beobachtet. Das Vergleichsmittel wies folgende Bestandteile auf:

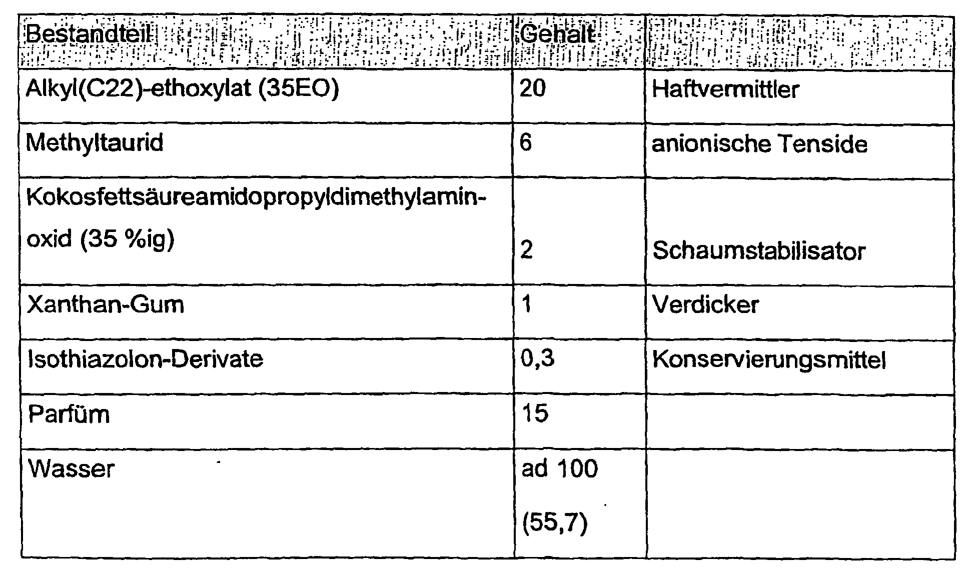

Die Viskosität beträgt circa 110.000 mPas bei 20° C (Haake-Viskosimeter, System Platte-Kegel, Sensor PK 1°, Schergefälle 25 s⁻¹).

Als Methyltaurid wurde Hostapon T 50%ig der Firma Clariant, als Kokosfettsäureamidopropyldimethylaminoxid (35 %ig) das Rewominox B 204 der Firma Rewo/Goldschmidt, als Xanthan-Gum das Rhodopol T der Firma Rhodia und als Parfüm das Citrix II 98-1103 der Firma Quest International eingesetzt.

Bei den Vergleichsversuchen mit den bekannten Mitteln wurden bei Auftrag von 5 ml Masse eines circa 1,3 cm breiten Strangs Spülzahlen von bis zu 300 Spülungen ermittelt, das heißt, daß das bekannte Mittel relativ hohe Spülzahlen aufweist und zur Erreichung einer hinreichenden Beduftung relativ hohe Parfümkonzentrationen, in dem Vergleichsversuch in Höhe von 15 Gew.%, erforderlich sind.

Die Schaumzahlen des bekannten Mittels wurden zu den obigen Bedingungen zu 60/60/50 ml bestimmt.

Die Oberfläche des bekannten Mittels wurden bei niedrigen Spülfrequenzen, das heißt, bei vier Spülungen/Tag, grieselig, uneben und zerfurcht. Bei längerer Standzeit ohne Spülen verhärtete die Oberfläche des aufgetragenen haftenden Sanitärmittels, sie wurde weiß und es bildete sich ein schwer löslicher Rest.

## Patentansprüche

1. Sanitärmittel zum Reinigen und/oder Desinfizieren und/oder zur Duftstoffabgabe, welches Mittel unmittelbar auf dem Sanitärgegenstand applizierbar ist, dort haftet und erst nach einer größeren Anzahl von Spülvorgängen abspülbar ist und welches Mittel
- Wasser,
- anionische und/oder nichtionische und/oder amphotere Tenside und
- einen Haftvermittler aus der aus Polyalkoxyalkanen, Alginaten, Diurethanen, Gelatine, Pectinen, Oleylaminen, Alkyldimethylaminoxiden, Stearaten, Sulfonaten, Sulfaten und Carbonaten bestehenden Gruppe umfaßt,
- wobei die Viskosität des Mittels wenigstens 15.000 mPas, gemessen mit einem Haake-Viskosimeter, System Platte-Kegel, Sensor PK 5 1°, gemessen bei einem Schergefälle von 25 s⁻¹ und 20°C, beträgt
**dadurch gekennzeichnet, daß** das Mittel zusätzlich
- entweder wenigstens eine aus der Gruppe Glycerin, 1,3-Dihydroxypropan, 1,3- oder 1,4- Dihydroxybutan, 1,3-Dihydroxyisobutan, Pentaerythrit aus gewählte Verbindung
- oder zwischen 1 und 20 Gew.%, vorzugsweise zwischen 5 und 15 Gew.% und besonders bevorzugt zwischen 7 und 13 Gew.% einer aliphatischen Di-, Oligo- oder Polyhydroxyverbindung oder deren Ether umfaßt.

2. Mittel nach Anspruch 1, **dadurch gekennzeichnet, daß** die aliphatische Di-, Oligo- oder Polyhydroxyverbindung oder deren Ether eine unverzweigte oder verzweigte und/oder geradzahlige oder ungeradzahlige aliphatische Verbindung ist.

3. Mittel nach Anspruch 1 oder 2, **dadurch gekennzeichnet, daß** die aliphatischen Di-, Oligo- oder Polyhydroxiverbindungen oder deren Ether Glycol, 1,2-Dihydroxypropan, 1,2- oder 2,3- Dihydroxibutan, 1,2-Dihydroxiisobutan, Diethylenglycol, Triethylenglycol, Polymethylen- oder Polyethylenglycol ist.

4. Mittel nach Anspruch 1, **dadurch gekennzeichnet, daß** das Mittel zwischen 1 und 20 Gew.%, vorzugsweise zwischen 5 und 15 Gew.% und besonders bevorzugt zwischen 7 und 13 Gew.% der Verbindung(en) aus der Gruppe Glycerin, 1,3-Dihydroxypropan, 1,3- oder 1,4- Dihydroxybutan, 1,3-Dihydroxyisobutan, Pentaerythrit umfaßt.

5. Mittel nach einem der Ansprüche 1 bis 3, **dadurch gekennzeichnet, daß** die Polyalkoxygruppe der Polyalkoxyalkane eine Polyethoxy-, Polypropoxy- oder Polybutoxy- oder auch eine gemischte Polyalkoxygruppe wie eine Poly(ethoxypropoxy)gruppe ist, wobei Polyethoxygruppen mit zwischen 15 und 55 Ethoxygruppen, insbesondere mit zwischen 25 und 45 und insbesondere mit 35+/-5 Ethoxygruppen, bevorzugt sind und/oder der Alkylrest wenigstens 12, vorzugsweise zwischen 16 und 30 Kohlenstoffatome aufweist.

6. Sanitärmittel nach einem der Ansprüche 1 bis 4, **dadurch gekennzeichnet, daß** die Konzentration des Haftvermittlers zwischen 10 und 40 Gew.%, vorzugsweise zwischen 15 und 35 Gew.% und besonders bevorzugt zwischen 20 und 30 Gew.%, beträgt.

7. Sanitärmittel nach einem der Ansprüche 1 bis 5, **dadurch gekennzeichnet, daß** der Wasseranteil in dem Mittel wenigstens 20 Gew.%, vorzugsweise zwischen 40 und 70 Gew.% und besonders bevorzugt zwischen 45 und 65 Gew.%, beträgt.

8. Sanitärmittel nach einem der Ansprüche 1 bis 6, **dadurch gekennzeichnet, daß** das Mittel etwa 1 Gew.% bis 25 Gew.%, insbesondere 5 bis 15 Gew.%, Duftstoffe, 1 Gew.% bis 20 Gew.%, insbesondere 5 bis 10 Gew.%., anionische Tenside, 20 Gew.% bis 40 Gew.% Haftvermittler, zwischen 1 und 20 Gew.%, vorzugsweise zwischen 5 und 15 Gew.%, der aliphatischen Di-, Oligo- oder Polyhydroxyverbindungen oder deren Ether oder der Verbindung(en) aus der Gruppe Glycerin, 1,3-Dihydroxypropan, 1,3- oder 1,4-Dihydroxybutan, 1,3-Dihydroxyisobutan, Pentaerythrit, 0 Gew.% bis 1 Gew.% Verdicker und 0 Gew.% bis 5 Gew.%, vorzugsweise zwischen 0 und 1 Gew.%, Konservierungsmittel sowie etwa 40 bis 70 Gew.% Wasser umfaßt.

9. Mittel nach einem der Ansprüche 1 bis 7, **dadurch gekennzeichnet, daß** das Mittel ein festes oder pastöses Sanitärmittel ist.

10. Sanitärmittel nach einem der Ansprüche 1 bis 8, **dadurch gekennzeichnet, daß** es in Form eines Gels vorliegt.

11. Sanitärmittel nach einem der Ansprüche 1 bis 9, **dadurch gekennzeichnet, daß** die Viskosität wenigstens 50.000 mPas, vorzugsweise wenigstens 90.000 mPas und besonders bevorzugt wenigstens 110.000 mPas, beträgt.

12. Verfahren zur Applikation eines Sanitärmittels nach einem der Ansprüche 1 bis 10, **dadurch gekennzeichnet, daß** das Mittel direkt auf die Oberfläche des zu reinigenden Sanitärgegenstandes aufgebracht wird und dort haftet.

## Claims

1. Sanitary product for cleaning and/or disinfecting and/or for deodorizing, which product can be applied directly to the sanitary ware, adheres there and is only washed off after a large number of flushing operations and which product comprises
- water,
- anionic and/or nonionic and/or amphoteric surfactants and
- an adhesion promoter from the group consisting of polyalkoxyalkanes, alginates, diurethanes, gelatines, pectins, oleylamines, alkyldimethylamine oxides, stearates, sulphonates, sulphates and carbonates,
- where the viscosity of the product is at least 15 000 mPas, measured using a Haake viscosimeter, plate-cone system, sensor PK 5 1°, measured at a shear drop of 25 s⁻¹ and 20°C,
**characterized in that** the product additionally comprises
- either at least one compound chosen from the group consisting of glycerol, 1,3-dihydroxypropane, 1,3- or 1,4-dihydroxybutane, 1,3-dihydroxyisobutane, pentaerythritol
- or between 1 and 20% by weight, preferably between 5 and 15% by weight and particularly preferably between 7 and 13% by weight of an aliphatic di-, oligo- or polyhydroxy compound or ethers thereof.

2. Product according to Claim 1, **characterized in that** the aliphatic di-, oligo- or polyhydroxy compound or ethers thereof is an unbranched or branched and/or even-numbered or odd-numbered aliphatic compound.

3. Product according to Claim 1 or 2, **characterized in that** the aliphatic di-, oligo- or polyhydroxy compounds or ethers thereof is glycol, 1,2-dihydroxypropane, 1,2- or 2,3-dihydroxybutane, 1,2-dihydroxyisobutane, diethylene glycol, triethylene glycol, polymethylene glycol or polyethylene glycol.

4. Product according to Claim 1, **characterized in that** the product comprises between 1 and 20% by weight, preferably between 5 and 15% by weight and particularly preferably between 7 and 13% by weight of the compound(s) from the group consisting of glycerol, 1,3-dihydroxypropane, 1,3- or 1,4-dihydroxybutane, 1,3-dihydroxyisobutane and pentaerythritol.

5. Product according to one of Claims 1 to 3, **characterized in that** the polyalkoxy group of the polyalkoxyalkanes is a polyethoxy, polypropoxy or polybutoxy group or else a mixed polyalkoxy group such as a poly(ethoxypropoxy) group, where polyethoxy groups having between 15 and 55 ethoxy groups, in particular having between 25 and 45 and in particular having 35 +/- 5 ethoxy groups, are preferred and/or the alkyl radical has at least 12, preferably between 16 and 30, carbon atoms.

6. Sanitary ware according to one of Claims 1 to 4, **characterized in that** the concentration of the adhesion promoter is between 10 and 40% by weight, preferably between 15 and 35% by weight and particularly preferably between 20 and 30% by weight.

7. Sanitary product according to one of Claims 1 to 5, **characterized in that** the water fraction in the product is at least 20% by weight, preferably between 40 and 70% by weight and particularly preferably between 45 and 65% by weight.

8. Sanitary product according to one of Claims 1 to 6, **characterized in that** the product comprises about 1% by weight to 25% by weight, in particular 5 to 15% by weight, of fragrances, 1% by weight to 20% by weight, in particular 5 to 10% by weight, of anionic surfactants, 20% by weight to 40% by weight of adhesion promoters, between 1 and 20% by weight, preferably between 5 and 15% by weight, of the aliphatic di-, oligo- or polyhydroxy compounds or ethers thereof or of the compound(s) from the group consisting of glycerol, 1,3-dihydroxypropane, 1,3- or 1,4-dihydroxybutane, 1,3-dihydroxyisobutane, pentaerythritol, 0% by weight to 1% by weight of thickener and 0% by weight to 5% by weight, preferably between 0 and 1% by weight, of preservative, and also about 40 to 70% by weight of water.

9. Product according to one of Claims 1 to 7, **characterized in that** the product is a solid or pasty sanitary product.

10. Sanitary product according to one of Claims 1 to 8, **characterized in that** it is in the form of a gel.

11. Sanitary product according to one of Claims 1 to 9, **characterized in that** the viscosity is at least 50 000 mPas, preferably at least 90 000 mPas and particularly preferably at least 110 000 mPas.

12. Method of applying a sanitary product according to one of Claims 1 to 10, **characterized in that** the product is applied directly to the surface of the sanitary ware to be cleaned and adheres there.

## Revendications

1. Agent sanitaire en vue du nettoyage et/ou de la désinfection et/ou de la libération de substances odorantes, lequel agent peut être appliqué immédiatement sur l'article sanitaire, adhère à ce dernier et ne peut en être éliminé par rinçage qu'après un grand nombre de procédures de rinçage et lequel agent comprend
- de l'eau,
- des agents tensioactifs amphotères et/ou anioniques et/ou non ioniques et
- un agent promoteur de l'adhésion fait à partir du groupe constitué de polyalkoxy alcanes, d'alginates, de diuréthanes, le gélatine, de pectines, d'oléylamines, d'alkyldiméthylamino-oxydes, de stéarates, de sulfonates, de sulfates et de carbonates,
- la viscosité de l'agent étant d'au moins 15 000 mPas, en cas de mesure à l'aide d'un viscosimètre Haake, à système plaque-cône, à détecteur PK 5 1 °, avec un gradient de cisaillement de 25 s⁻¹ et à la température de 20° C,
**caractérisé en ce que** l'agent comprend en outre
- ou moins un compose sélectionné parmi le groupe glycérine, 1,3-dihydroxypropane, 1,3- ou 1,4-dihydroxybutane, 1,3-dihydroxyisobutane, pentaérythritol
- ou entre 1 et 20 % en poids, de préférence, entre 5 et 15 % en poids et, en particulier , de préférence, entre 7 et 13 % en poids d'un composé aliphatique di-, oligo- ou polyhydroxy ou de ses éthers.

2. Agent selon la revendication 1, **caractérisé en ce que** le composé aliphatique di-, oligo- ou polyhydroxy ou ses éthers est un composé aliphatique non ramifié et/ou ramifié et/ou pair et/ou impair.

3. Agent selon la revendication 1 ou 2, **caractérisé en ce que** le composé aliphatique di-, oligo- ou polyhydroxy ou ses éthers est le glycol, le 1,2-dihydroxypropane, le 1,2-ou 2,3-dihydroxybutane, le 1,2-dihydroxyisobutane, le diéthylèneglycol, le triéthylèneglycol, le polyméthylèneglycol ou le polyéthylèneglycol.

4. Agent selon la revendication 1, **caractérisé en ce que** l'agent comprend entre 1 et 20 % en poids, de préférence, entre 5 et 15 % en poids et, en particulier, de préférence, entre 7 et 13 % en poids du ou des composé (s) du groupe glycérine, 1,3-dihydroxypropane, 1,3- ou 1,4-dihydroxybutane, 1,3-dihydroxyisobutane, pentaérythritol.

5. Agent selon l'une quelconque des revendications 1 à 3, **caractérisé en ce que** le groupement polyalkoxy des polyalkoxy alcanes est un groupement polyéthoxy, polypropoxy ou polybutoxy ou également un groupement polyalkoxy mixte comme un groupement poly(éthoxypropoxy), les groupements polyéthoxy avec entre 15 et 55 groupements éthoxy, en particulier entre 25 et 45 et, en particulier 35 +/- 5 groupements éthoxy étant préférés et/ou le résidu alkyle présentant au moins 12, de préférence, entre 16 et 30 atomes de carbone.

6. Agent sanitaire selon l'une quelconque des revendications 1 à 4, **caractérisé en ce que** la concentration de l'agent promoteur de l'adhésion est comprise entre 10 et 40 % en poids, de préférence, entre 15 et 35 % en poids et, en particulier, de préférence, entre 20 et 30 % en poids.

7. Agent sanitaire selon l'une quelconque des revendications 1 à 5, **caractérisé en ce que** la proportion d'eau dans l'agent est d'au moins 20 % en poids, de préférence, est compris entre 40 et 70 % en poids, et, en particulier, de préférence, est compris entre 45 et 65 % en poids.

8. Agent sanitaire selon l'une quelconque des revendications 1 à 6, **caractérisé en ce que** l'agent comprend environ de 1 % en poids à 25 % en poids, en particulier de 5 à 15 % en poids de substances odorantes, de 1 % en poids à 20 % en poids, en particulier de 5 à 10 % en poids d'agents tensioactifs anioniques, de 20 % en poids à 40 % en poids d'agents de promoteur de l'adhésion, entre 1 et 20 % en poids, de préférence, entre 5 et 15 % en poids de composés aliphatiques di-, oligo- ou polyhydroxy ou de leurs éthers ou d'un ou de composé(s) du groupe glycérine, 1,3-dihydroxypropane, 1,3- ou 1,4- dihydroxybutane, 1,3-dihydroxyisobutane, pentaérythritol, de 0 % en poids à 1 % en poids d'agent épaississant et de 0 % en poids à 5 % en poids, de préférence, entre 0 et 1 % en poids d'agents de conservation ainsi que d'environ 40 à 70 % en poids d'eau.

9. Agent selon l'une quelconque des revendications 1 à 7, **caractérisé en ce que** l'agent est un agent sanitaire solide ou pâteux.

10. Agent sanitaire selon l'une quelconque des revendications 1 à 8, **caractérisé en ce qu'**il est présent sous la forme d'un gel.

11. Agent sanitaire selon l'une quelconque des revendications 1 à 9, **caractérisé en ce que** la viscosité est d'au moins 50 000 mPas, de préférence, d'au moins 90 000 mPas et, en particulier, de préférence, d'au moins 110 000 mPas.

12. Procédé en vue de l'application d'un agent sanitaire selon l'une quelconque des revendications 1 à 10, **caractérisé en ce que** l'agent est directement appliqué sur la surface de l'article sanitaire à nettoyer et **en ce qu'**il adhère à ce dernier.
